# EUROPEAN PATENT APPLICATION

(11) **EP 3 527 242 A1**
(43) Date of publication of application: **21.08.2019**
(21) Application number: 18157250.4
(22) Date of filing: 16.02.2018
(51) Int. Cl.: A61M 5/168

(54) **INFUSION STATUS INDICATOR OF A DRUG DELIVERY DEVICE**

(71) Applicant: Debiotech S.A., 1004 Lausanne (CH)
(72) Inventor: CHAPPEL, Eric, 1004 Lausanne (CH); DUMONT-FILLON, Dimitry, 1004 Lausanne (CH)
(74) Representative: Weihs, Bruno Konrad

(57) **Abstract**

The present invention relates to an indicator device which comprises one or several plungers inside an elongated cavity. Said indicator device, is intended to provide to the user at least one information related to the current status of a delivery device. The indicator device comprises at least one plunger configured to reach pre-determined positions in response to the status of the fluid flow through the fluid pathway.

## Description

### FIELD OF INVENTION

The present invention discloses an indicator device configured to provide information to a user about the infusion status of a delivery device for example a drug delivery device which may be configured to infuse a single bolus.

### STATE OF THE ART

New therapies developed by pharmaceutical companies require a new generation of delivery device. Said new generation of delivery device has to allow the infusion of a determined amount of fluid to a patient over a time period. Some therapies may require infusing the entire content of the reservoir into a single bolus.

Preferentially, the delivery device can only be used once. It is therefore necessary to develop a device that could address the new requirements.

This document describes various embodiments of a smart and cheap indicator device for a delivery device and other embodiments comprising, for example, a valve device.

### GENERAL DESCRIPTION OF THE INVENTION

The present invention relates to an indicator device which comprises a single plunger or several plungers configured to indicate a status of the infusion. The present invention further relates to a valve allowing the occlusion of a fluid pathway.

One of the goals of the invention is to present directly or indirectly, to a user, at least one information (preferentially at least two, more preferentially three) related to the current status of delivery device. Preferentially, the device is configured to provide a discrete signal (for example a visual signal) indicating a fluid flow condition.

The indicator device comprises at least one element (for example a plunger) configured to reach determined positions in response to the status of the fluid flow through the fluid pathway.

According to a first aspect, the invention provides an indicator device for indicating an infusion status of a medical device adapted to deliver a solution to a patient. The indicator device may include a body, an elongated cavity arranged into the body and having a first end and a second end, and at least two plungers (a first plunger and a second plunger; optionally an additional plunger) configured to move relative to the elongated cavity. Preferentially, the first plunger and the second plunger are arranged into the elongated cavity in such a manner to define at least one variable volume, for example three variable volumes:
- a first variable volume formed between the first end of the elongated cavity and the first plunger,
- a second variable volume formed between the first plunger and the second plunger, and
- a third variable volume formed between the second plunger and the second end of the elongated cavity.
The first plunger may be configured to move depending on the pressure of the solution intended to be delivered.

The first plunger may provide a fluid-tight wall between the first variable volume and the second variable volume.

The first variable volume, the second variable volume and the third variable volume may comprise a fluid such as a liquid or a gas for example air or compressible fluid. The first variable volume may be configured to receive a volume fraction of the solution which may be expelled from the first variable volume at the end of the infusion.

The first plunger may perform forward and backward motion.

The first plunger (or the first variable volume) may be in pressure communication with the solution intended to be infused to the patient and a pressure gradient or a pressure variation of this solution may cause or allow the first plunger motion.

For example, the first plunger (or the first variable volume) may be in pressure communication with a reservoir storing the solution intended to be infused. This reservoir may be a pressurized reservoir. The medical device may comprise the reservoir and a housing in which the reservoir is arranged.

The first end of the cavity may comprise a fluid port in fluid communication with the solution intended to be delivered. For example, the fluid port may be in fluid communication with the reservoir storing the solution or with the fluid pathway between the internal compartment of the reservoir and an outlet of the medical device.

The second plunger may move depending on the first plunger. For example, the first plunger may push the second plunger from a position to a determined position which may be visible or hidden to the user. In this purpose, the indicator device may comprise one or more windows in order to render visible at least one of the first plunger and the second plunger in determined position(s).

The second plunger may comprise a through hole configured to provide a fluid communication between the second variable volume and the third variable volume.

The indicator device may allow a discrete visual signal indicating a fluid flow condition depending on the position of at least one of the first plunger and the second plunger. Depending on the position of the plungers, the plungers may be visible or hidden to the user, in order to indicate a status of the medical device such as ready to inject, infusion on-going or end of infusion.

The elongated cavity may further comprise a mechanical stop (also called stop member) arranged between the first end and the second end in order to maintain at least one of the first plunger and the second plunger in a determined position.

The second end may comprise a vent in order to provide a pressure equilibration of the third variable volume with the outside environment.

The indicator device may further comprise a biasing device arranged between the first plunger and the second plunger.

According to a second aspect, the invention provides an indicator device for indicating an infusion status of a medical device adapted to deliver a solution to a patient. The indicator device may include a body, an elongated cavity arranged into the body and having a first end and a second end, and a first plunger configured to move relative to the elongated cavity. Preferentially the first plunger is arranged into the elongated cavity and may be configured to have at least one (stable) position. More preferentially, the first plunger may be configured to have at least two distinct positions, for example three distinct positions: a first initial position, a second position, and a third position.

The first plunger may be configured to move depending on the pressure of the solution intended to be delivered.

The first plunger may perform forward and backward motion.

The first plunger may provide a fluid-tight wall between a first variable volume and a second variable volume. The first variable volume may be defined between the first end of the cavity and the first plunger and the second variable volume may be defined between the first plunger and an other end of the cavity, for example an opposite end of the first end, which may be the second end.

The first plunger (or the first variable volume) may be in pressure communication with the solution intended to be infused to the patient and a pressure gradient or a pressure variation of this solution may cause or allow the first plunger to move.

For example, the first plunger (or the first variable volume) may be in pressure communication with a reservoir storing the solution intended to be infused. This reservoir may be a pressurized reservoir. The medical device may comprise the reservoir and a housing in which the reservoir is arranged.

The first end of the cavity may comprise a fluid port in fluid communication with the solution intended to be delivered. For example, the fluid port may be in fluid communication with the reservoir storing the solution or with the fluid pathway between the internal compartment of the reservoir and an outlet of the medical device.

The indicator device may allow a discrete visual signal indicating a fluid flow condition depending on the position of the first plunger. Depending on the position of the first plunger, the plunger may be visible or hidden to the user, in order to indicate a status of the medical device such as ready to inject, infusion on-going or end of infusion. In this purpose, the indicator device may comprise one or more windows in order to render visible the first plunger in at least one of the first initial position, the second position and the third position.

The elongated cavity may further comprise a mechanical stop (also called stop member) arranged between the first end and the second end in order to maintain the first plunger in a determined position (the first initial position, the second initial position, or the third position).

The second end may comprise a vent in order to provide a pressure equilibration of the second variable volume with the outside environment.

The indicator device may further comprise a biasing device arranged between the first plunger and the second end of the cavity.

The indicator may comprise a second plunger which may be arranged into the cavity between the first plunger and the second end of the cavity. The second plunger may move depending on the first plunger (for example depending on the plunger motion according to a determined way/direction). For example, the first plunger may push the second plunger from a position to a determined position which may be visible or hidden to the user. For this purpose, the indicator device may comprise one or more windows in order to render visible at least one of the first plunger and the second plunger in determined position(s).

The second plunger may comprise a through hole configured to provide a fluid communication between the volume defined between the first plunger and the second plunger and the other volume defined between the second plunger and the second end.

According to a third aspect, the invention provides an indicator device indicating an infusion status of a medical device adapted to deliver a solution to a patient. The indicator device may include:
- a body having a window and an elongated cavity arranged into the body and having a first end and a second end,
- a first plunger configured to provide a visual signal through the window and to move relative to the elongated cavity, and
- a compressible visual element configured to provide a visual signal through the window.

Preferentially, the first plunger and the compressible visual element are arranged into the elongated cavity. The compressible visual element may be configured to be compressed into the cavity between the second end of the cavity and the first plunger when the plunger moves toward the second end of the cavity.

The first plunger may be configured to move depending on the pressure of the solution intended to be delivered.

The first plunger may perform forward and backward motion and the compressible visual element may be used as a biasing means which exerts an opposite force to the solution pressure against the first plunger.

The first plunger (or the first variable volume) may be in pressure communication with the solution intended to be infused to the patient and a pressure gradient or a pressure variation of this solution may cause or allow the first plunger motion.

For example, the first plunger (or the first variable volume) may be in pressure communication with a reservoir storing the solution intended to be infused. This reservoir may be a pressurized reservoir. The medical device may comprise the reservoir and a housing in which the reservoir is arranged.

The first end of the cavity may comprise a fluid port in fluid communication with the solution intended to be delivered. For example, the fluid port may be in fluid communication with the reservoir storing the solution or with the fluid pathway between the internal compartment of the reservoir and an outlet of the medical device.

The indicator device may allow a discrete visual signal indicating a fluid flow condition depending on the position of at least one of the first plunger and the compressible visual element. Depending on the position of the plunger and the compressible visual element, the plunger and/or the compressible visual element may be visible or hidden to the user, in order to indicate a status of the medical device such as ready to inject, infusion on-going or end of infusion. When the medical device is in a first status, the window may allow seeing the compressible visual element, for example the user can see an orange color indicating that the medical device is ready to inject. When the medical device is in a second status, the window may allow seeing a wall of the cavity, for example the user can see a green color indicating that the solution is flowing to the patient. When the medical device is in a third status, the window may allow seeing the plunger, for example the user can see a blue color indicating that the infusion is ended.

The elongated cavity may further comprise a mechanical stop arranged between the first end and the second end in order to maintain at least one of the first plunger and the compressible visual element at a determined position.

The second end may comprise a vent in order to provide a pressure equilibration (of the volume between the second end and an end of the compressible visual element or of the first plunger) with the outside environment.

The indicator device may further comprise a biasing device. The biasing means may be a gas trapped between the first plunger and the second end or the compressible visual element.

The compressible visual element may be a plunger having an elongated body which is configured to fold when it is compressed.

According to a fourth aspect, the invention provides a medical device adapted to deliver a solution to a patient. The medical device may include an indicator device, a pressurized reservoir to store the solution, an outlet port in fluid communication with the patient, and a fluid pathway from the interior of the pressurized reservoir to the outlet port.

The indicator device may be one of the described indicator device of the invention. For example, the indicator device may comprise an elongated cavity arranged into the medical device and having a first end and a second end, and a first plunger configured to move relative to the elongated cavity.

Preferentially, the indicator device is in pressure communication with the fluid pathway.

The first plunger may be configured to move depending on the pressure of the solution stored in the pressurized reservoir or the fluid present into a part of the fluid pathway.

The first plunger may perform forward and backward motion.

The first plunger may provide a fluid-tight wall between a first variable volume and a second variable volume. The first variable volume may be defined between the first end of the cavity and the first plunger and the second variable volume may be defined between the first plunger and an other end of the cavity, for example an opposite end of the first end, which may be the second end. The indicator device may comprises an additional plunger arranged into the cavity for example between the first plunger and the second end and may divide the second variable volume into a second variable volume and a third variable volume.

The first plunger (or the first variable volume) may be in pressure communication with the solution initially stored into the pressurized reservoir or present into a part of the fluid pathway. A pressure gradient or a pressure variation of this solution may cause or allow the first plunger motion.

Preferentially, the medical device comprises a housing in which may be arranged a part of the indicator device, a part of the reservoir, and a part of the fluid pathway.

The indicator device may allow a discrete visual signal indicating a fluid flow condition depending on the position of the plunger(s). Depending on the position of the plunger(s), the plunger(s) may be visible or hidden to the user, in order to indicate a status of the medical device such as ready to inject, infusion on-going or end of infusion. For this purpose, the indicator device and/or the medical device may comprise one or more windows in order to render visible the plunger(s) in at least one position.

One of the goals of the invention is to prevent any free flow before the activation of the delivery device, for example when the user fills the reservoir.

According to a fourth aspect, the invention provides a valve device arranged into a fluid pathway as described above. The valve device may be configured in order to allow the solution flow to the outlet when the pressurized reservoir or the solution pressure reaches a predetermined threshold.

Preferentially, the device does not need any power source such as battery or electronic device connected to the indicator device or the valve device. The (linear) movement is due to the pressure of the solution in the drug reservoir and/or in the fluid pathway (between the reservoir and the outlet device).

### LIST OF FIGURES

The present invention will be better understood at the light of the following detailed description which contains non-limiting examples illustrated by the following figures:

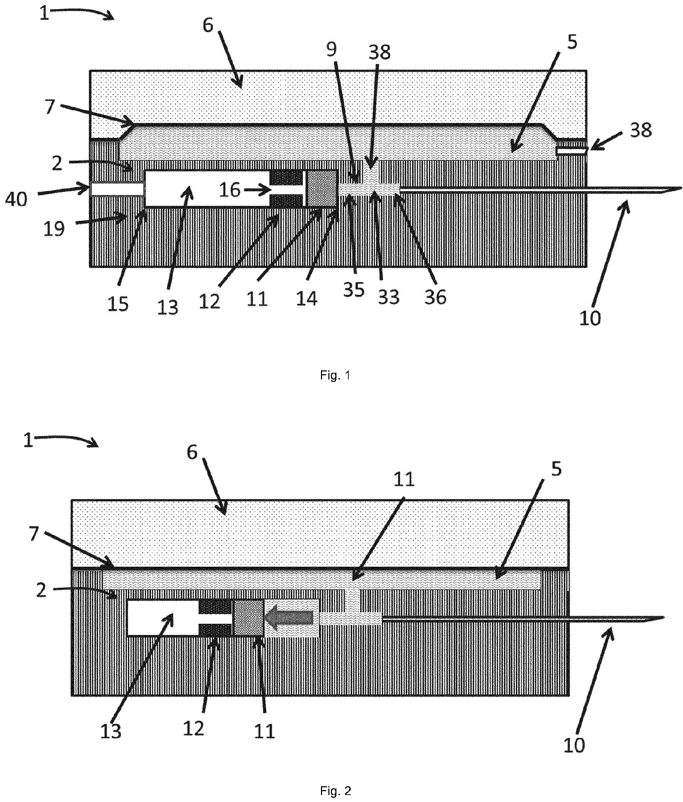

**Figure 1** shows an embodiment of the drug delivery device before injection, comprising a propellant reservoir (here liquefied gas), a drug reservoir tightly separated from the gas reservoir and an indicator device having two plungers.
**Figure 2** shows the same device (of the figure 1) after activation, the membrane between the propellant and drug reservoir transmitting the gas pressure to the drug. The two plungers are translated to the left part of the cylinder until the air pressure in the left part of the cylinder becomes substantially equal to the drug reservoir pressure.

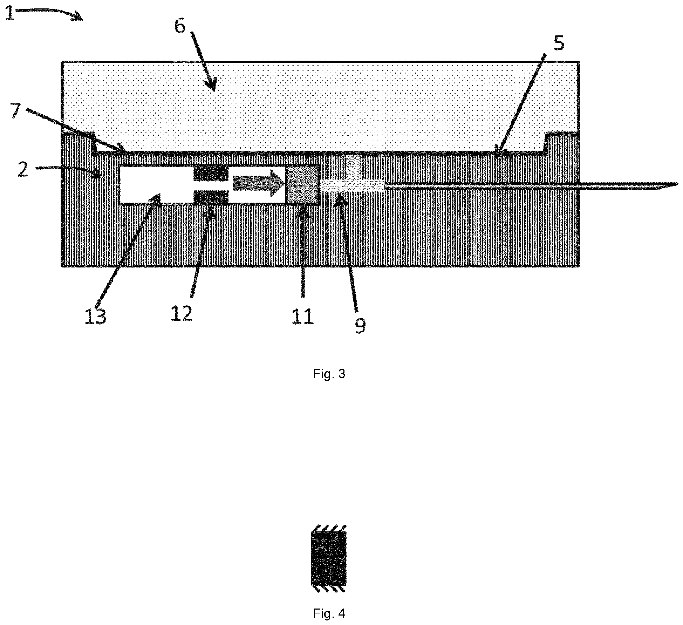

**Figure 3** shows the same device (of the figure 1) at the end of the injection, wherein the pressure in the communication port drops to zero, inducing a movement of the solid plunger toward its initial position.
**Figure 4** shows side-view of an example of a plunger with sealing lip at 45°.

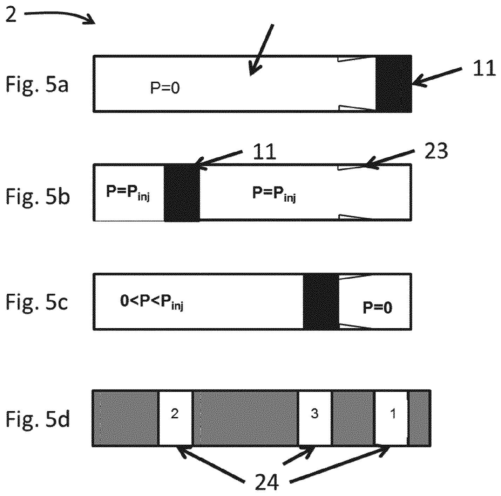

**Figures 5a, b, c, and d** show an embodiment wherein the indicator device comprises a single plunger having a mechanical stop (for example conical narrowing) which may be configured to allow the definition of three different plunger positions. The figure 5a shows a first position related to the status "ready to inject", the figure 5b shows a second position related to the status "infusion on-going", and figure 5c shows a third position related to the status "end of infusion". The figure 5d shows three windows placed in front of the infusion status indicator (or at least one) may be configured to allow the visualization of the plunger (bottom figure).

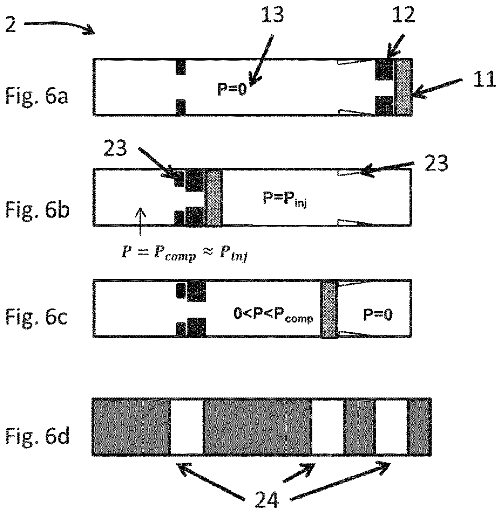

**Figures 6a, b, c, and d** show an embodiment wherein the indicator device comprises a first mechanical stop (left part of the cylinder) and a second mechanical stop (conical narrowing in the right part of the cylinder). The three well defined positions of the two plungers before injection (Fig. 6a), during injection (Fig. 6b) and after injection (Fig. 6c) are shown. One or more windows may be arranged in front of the infusion status indicator to allow the visualization of the plungers (Fig. 6d).

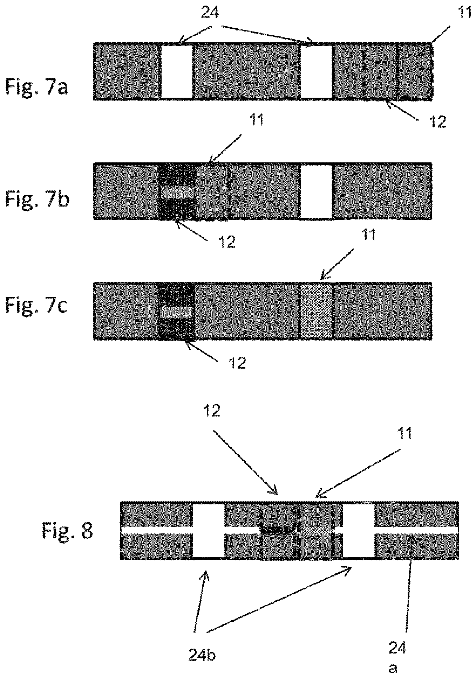

**Figures 7a, b, and c** show an embodiment wherein the indicator device comprises two plunger and two distinct windows. The second plunger may be red while the first plunger may be green. Before infusion no plunger is visible (Fig.7a). During infusion the second plunger is visible through the window located on the left part of the cylinder (Fig.7b). At the end of the infusion the first plunger becomes visible through the window located on the right part of the cylinder (Fig.7c).
**Figure 8** shows an embodiment wherein the dual plunger infusion status indicator comprises two vertical windows as well as a horizontal one. The plungers are in an intermediate position just after activation. The hole of the second plunger is not represented here for sake of clarity. The horizontal window is here useful to determine that the infusion has been activated since the plungers are no longer at their initial locations and not visible through at least one of the vertical windows.

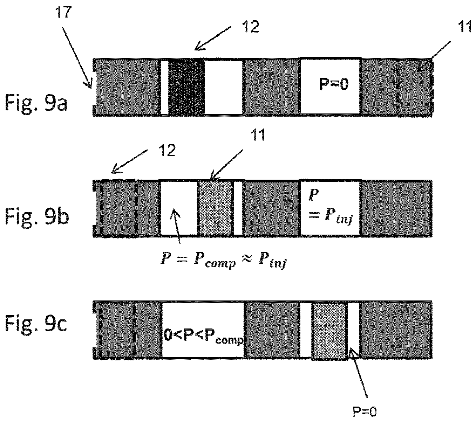

**Figures 9a, b, and c** show an embodiment wherein the indicator device comprises a vent device for example the cavity (the third variable volume) is open to air. Before infusion only the second plunger is visible (fig. 9a). During infusion the second plunger moves towards the end of the cavity while the first plunger becomes visible in the left window (fig. 9b). At the end of the infusion the first plunger becomes visible through the window located on the right part of the cylinder (fig. 9c).

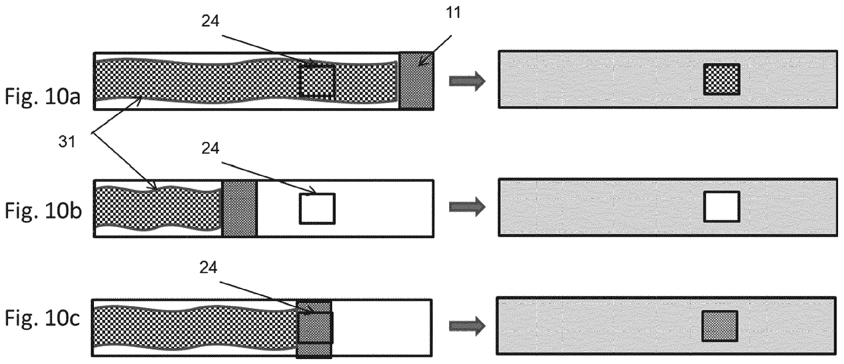

**Figures 10a, b, and c** show an example of an indicator device comprising a compressible visual element and a plunger.

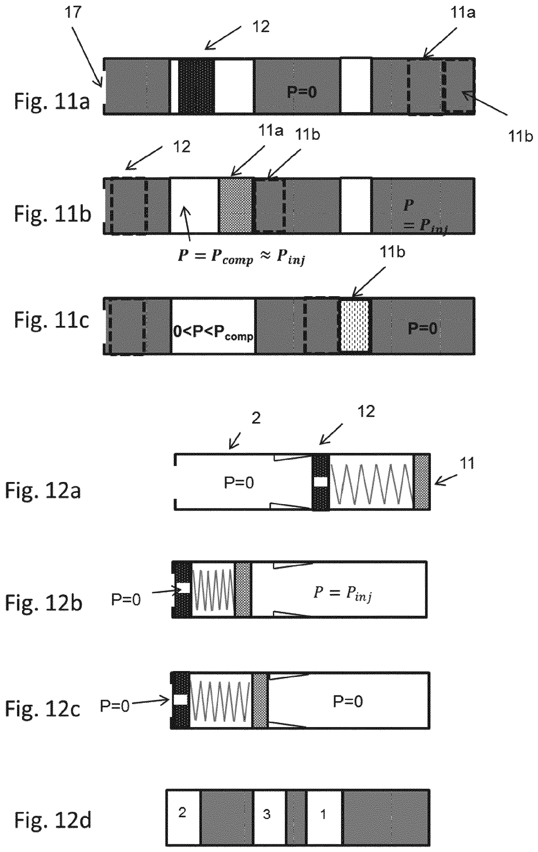

**Figures 11a, b, and c** show an embodiment wherein the indicator device comprises two windows and at least two plungers, and optionally wherein the cylinder is open to air. Before infusion only the second plunger is visible (fig. 11a). During infusion the second plunger moves towards the end of the cylinder while a first part of the first plunger becomes visible in the left window (fig. 11b). At the end of the infusion only a second part of the first plunger (or an additional plunger, for example a third plunger) is visible through the window located on the right part of the cylinder (fig. 11 c).
**Figures 12a, b, c, and d** show an embodiment wherein the indicator device comprises a biasing device (for example a spring) arranged between a first plunger and a second plunger. The three well defined positions of the two plungers before injection (fig. 12a), during injection (fig. 12b) and after injection (fig. 12c) are shown. Windows placed in front of the infusion status indicator may be configured to allow the visualization of the plungers (fig. 12d).

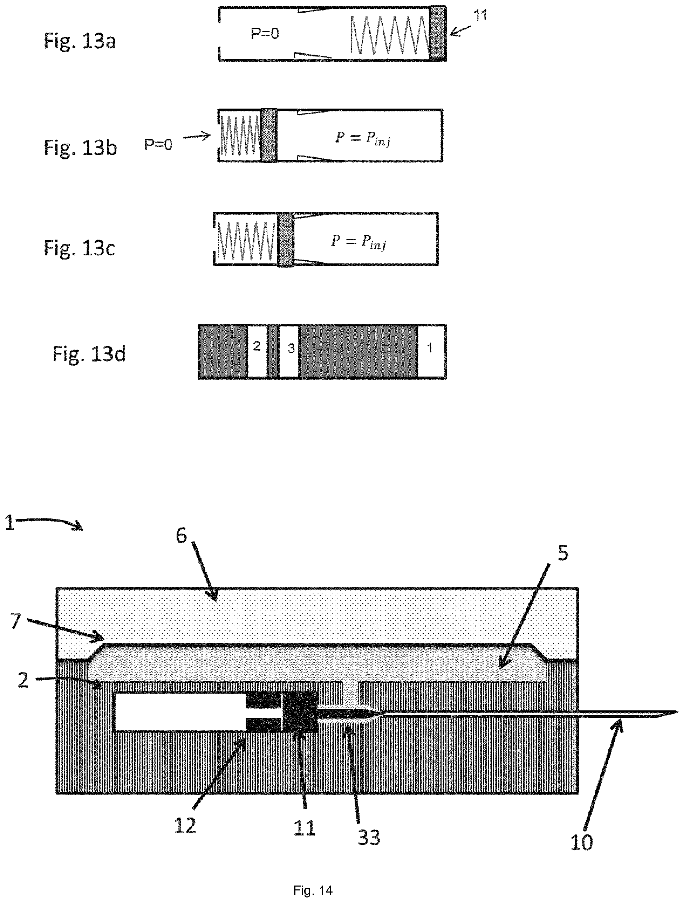

**Figures 13a****,** **b, c, and d** show an embodiment wherein the indicator device comprises a biasing device (for example a spring) and a single plunger. The three well defined positions of the plunger before injection (fig. 13a), during injection (fig. 13b) and after injection are shown (fig. 13c). Windows placed in front of the infusion status indicator may be configured to allow the visualization of the plungers (fig. 13d).
**Figure 14** shows an example of a drug delivery device before injection. Such drug delivery device may comprise a propellant reservoir (here liquefied gas), a drug reservoir tightly separated from the gas reservoir and an indicator device with a second plunger and a first plunger having a protrusion. The protrusion of the first plunger may be configured to block the flow as long as the pressure in the reservoir does not exceed a predefined value. The protrusion may be considered as a valve device of the fluid pathway.

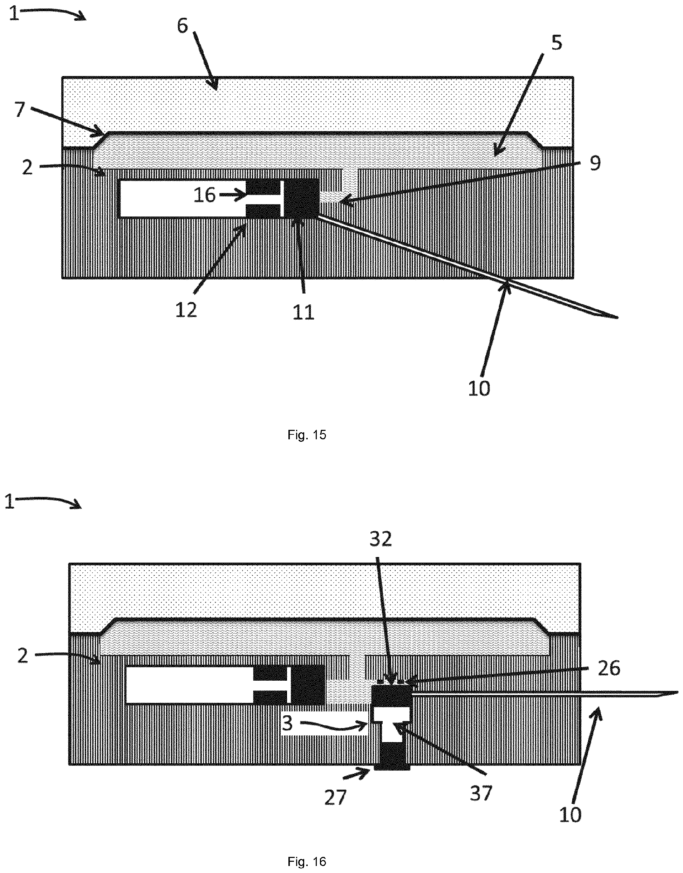

**Figure 15** shows an example of drug delivery device before injection comprising a propellant reservoir (here liquefied gas), a drug reservoir tightly separated from the gas reservoir, an indicator device having at least one plunger used as a valve device which blocks the flow as long as the pressure in the reservoir does not exceed a predefined value and a needle that is off-axis with the cylinder. In this example the first variable volume is a part of the fluid pathway or divides the fluid pathway in two distinct parts, a first part in fluid communication with the internal compartment of the reservoir and a second part in fluid communication with the outlet device (for example the needle).
**Figure 16** shows an example of drug delivery device before injection, comprising a propellant reservoir (here liquefied gas), a drug reservoir tightly separated from the gas reservoir, a needle, a valve device, and an indicator device. The indicator device comprises a first and a second plunger. The valve device comprises a plunger adapted to move inside a cavity and a plug. The pressure of the drug applies onto the top surface of this plunger thanks to the presence of plunger stoppers. The user may have to remove the plug to allow the plunger of the valve device to open the fluid pathway. In the embodiment the indicator device is optional.

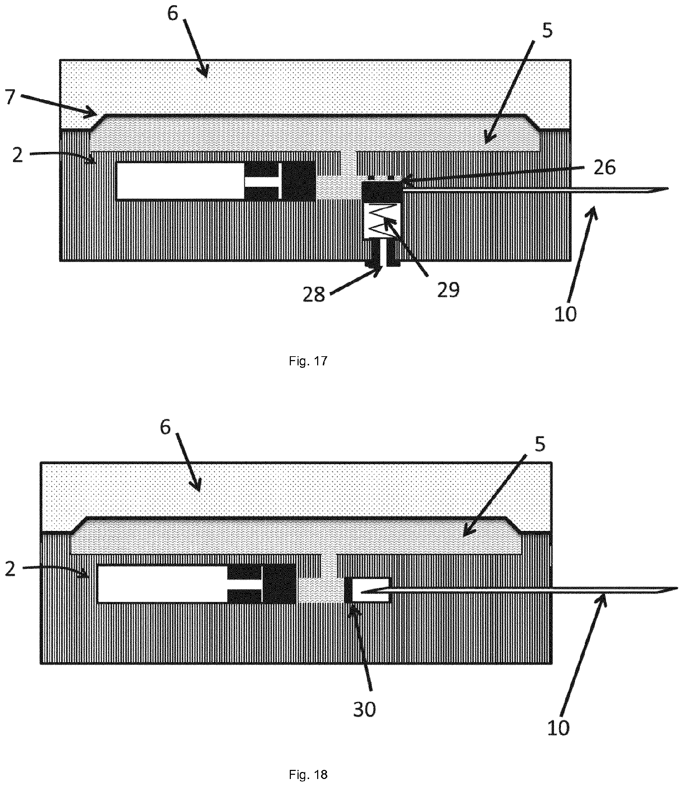

**Figure 17** shows an example of drug delivery device before injection, comprising a propellant reservoir (here liquefied gas), a drug reservoir tightly separated from the gas reservoir, a needle, an indicator device, and a valve device. The valve device may comprise a plunger inside a cavity in direct communication with the outlet device and a plug, said cavity comprising a spring and a vented and threaded adjustable plug. The pressure of the drug applies onto the top surface of the plunger thanks to the presence of plunger structuration. In the embodiment the indicator device is optional.
**Figure 18** shows an example of drug delivery device before injection, comprising a propellant reservoir (here liquefied gas), a drug reservoir tightly separated from the gas reservoir, an indicator device, and a valve device. The valve device comprises an elastomeric membrane used as crack valve and a double beveled needle (outlet device). In the embodiment the indicator device is optional.

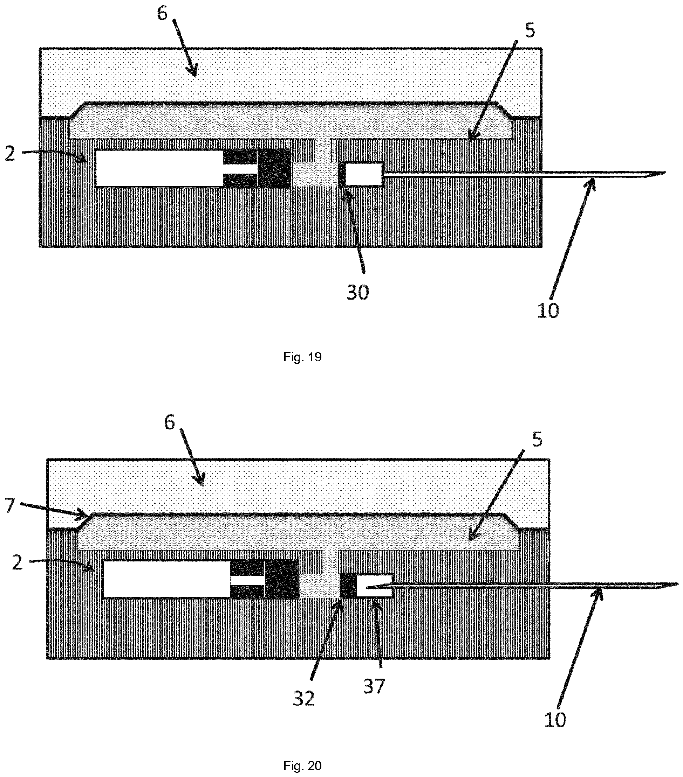

**Figure 19** shows an example of drug delivery device before injection, comprising a propellant reservoir (here liquefied gas), a drug reservoir tightly separated from the gas reservoir, an indicator device, and an elastomeric membrane used as crack valve and a needle. In the embodiment the indicator device is optional.
**Figure 20** shows an example of drug delivery device before injection, comprising a propellant reservoir (here liquefied gas), a drug reservoir tightly separated from the gas reservoir, a double beveled needle, an indicator device, and a valve device having a cylinder and a plunger being used as crack valve. In the embodiment the indicator device is optional. The plunger of the valve device is configured to move through the cylinder.

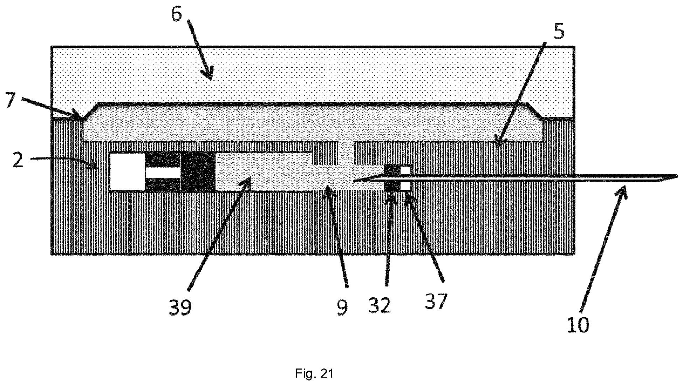

**Figure 21** shows an example of drug delivery device during injection, comprising a propellant reservoir (here butane at 20°C for example), a drug reservoir tightly separated from the gas reservoir, a double beveled needle, an indicator device, and a valve device having a cylinder and a plunger which has been pierced by the needle after activation of the device. In the embodiment the indicator device is optional.

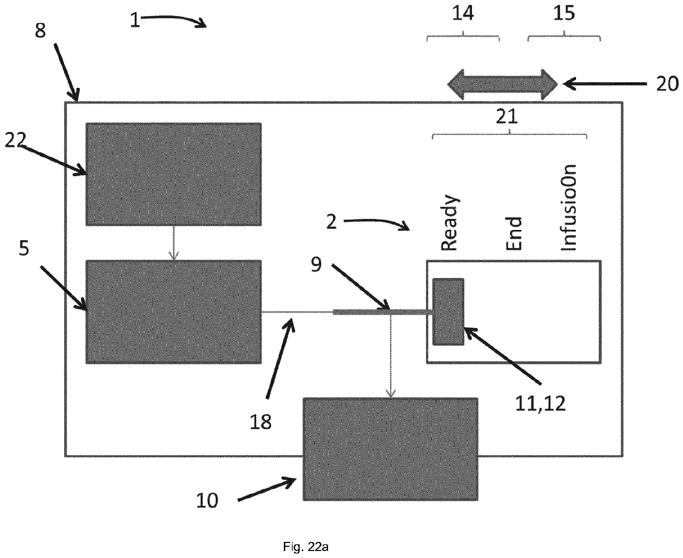

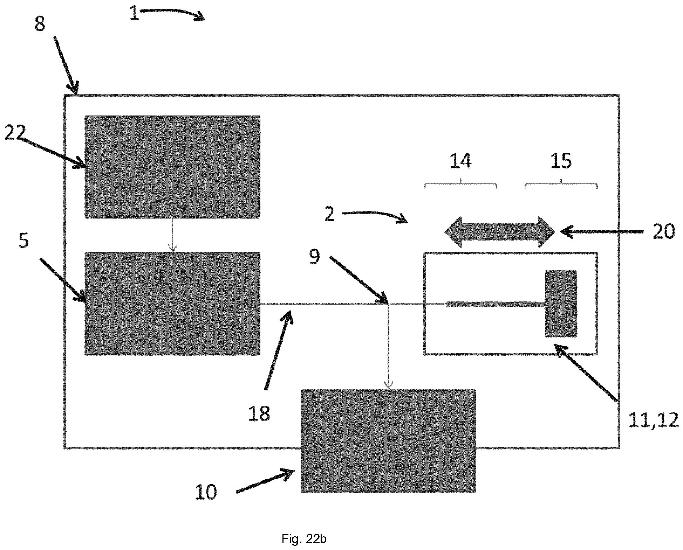

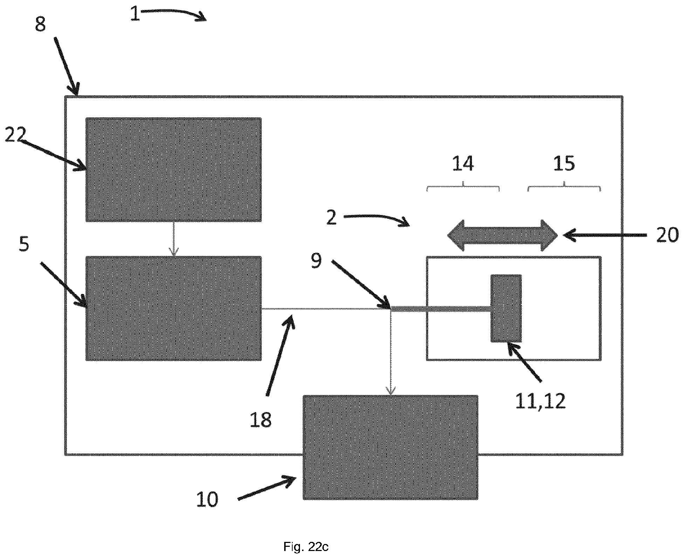

**Figures 22a****,** **b****, and** **c** show the general concept of the indicator device of the invention

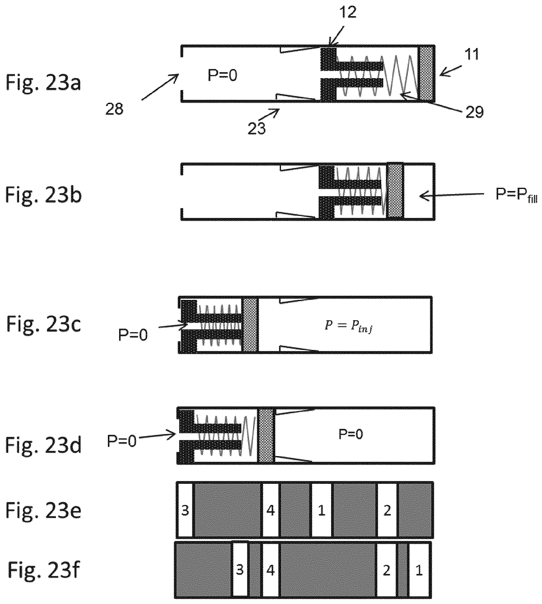

**Figures 23a, b, c, d, e, and f** show an other potential embodiment of the invention.

### LIST OF ELEMENTS

- 1: delivery device
- 2: indicator device
- 3: valve device
- 4: pressure device
- 5: drug reservoir
- 6: propellant reservoir
- 7: flexible or movable membrane
- 8: housing
- 9: communication port or fluid port
- 10: outlet device
- 11: first plunger
- 11a: first part of the first plunger
- 11b: second part of the first plunger
- 12: second plunger
- 13: cavity
- 14: first end of the cavity
- 15: second end of the cavity
- 16: through hole
- 17: venting device
- 18: fluid pathway
- 19: body
- 20: linear displacement of the plunger(s)
- 21: Visual inscription
- 22: pumping device
- 23: stop member / mechanical stop
- 24: window(s)
- 24a: horizontal window
- 24b: vertical window
- 25: spring/biasing element
- 26: plunger stopper
- 27: plug
- 28: vent
- 29: spring/biasing element
- 30: elastomeric membrane
- 31: compressible visual element
- 32: movable septum or plunger
- 33: T-sape channel
- 34: first fluid port
- 35: second fluid port
- 36: third fluid port
- 37: valve cavity
- 38: inlet port
- 39: solution to be delivered
- 40: vent (optional)

### DETAILED DESCRIPTION OF THE INVENTION

In the following detailed description, reference is made to the accompanying drawings that form a part hereof, and in which are shown by way of illustration several embodiments of devices, systems and methods. It is to be understood that other embodiments are contemplated and may be made without departing from the scope or spirit of the present disclosure. The following detailed description, therefore, is not to be taken in a limiting sense. The features described therein may be comprised in the different embodiments.

All scientific and technical terms used herein have meanings commonly used in the art unless otherwise specified. The definitions provided herein are to facilitate the understanding of certain terms used frequently herein and are not meant to limit the scope of the present disclosure.

As used in this specification and the appended claims, the singular forms "a", "an", and "the" encompass embodiments having plural referents, unless the content clearly dictates otherwise.

As used in this specification and the appended claims, any direction referred to herein, such as "top", "bottom", "left", "right", "upper", "lower", and other directions or orientations are described herein for clarity in reference to the figures and are not intended to be limiting of an actual device or system. Devices and systems described herein may be used in a number of directions and orientations.

As used herein, "have", "having", "include", "including", "comprise", "comprising" or the like are used in their open ended sense, and generally mean "including, but not limited to".

As used herein, "plunger" or the like are used in it open ended sense, and generally mean a movable piece arranged into a cavity and configured to (optionally tightly) separates said cavity in two distinct parts.

As used herein, "cylinder" or the like are used in it open ended sense, and generally mean a solid formed by a line that moves parallel to itself by leaning on a flat curve.

As used in this specification and the appended claims, the term "or" is generally employed in its sense including "and/or" unless the content clearly dictates otherwise.

### GENERAL OPERATING PRINCIPLE

The figures 22a, 22b and 22c show the general concept of the indicator device of the invention. The shape and the size are not representative. In these figures only one plunger is drawn but the basic concept stays identic for two plungers, thus both references are used for the plunger (11, 12).

The delivery device (1) comprises a reservoir (5) adapted to store a medical fluid (for example a drug or other), a pumping device (22) adapted to move the medical fluid stored in the reservoir, an outlet device (10) adapted to be in fluid communication with a patient and a fluid pathway (18) providing a fluid communication between the reservoir (5) and the outlet device (10).

In order to simplify the description, the reservoir (5) of the medical fluid is also called drug reservoir but this wording does not limit the type of medical fluid which may be stored in the reservoir.

The medical fluid reservoir (5) may comprise a flexible membrane (7) and/or a movable membrane (7). The drug reservoir may further comprise a rigid part. At least one of the membrane (7) and the rigid part define an internal compartment adapted to receive the medical fluid to be infused to the patient. The drug reservoir may further comprise an outlet. The outlet of the reservoir is in fluid communication with the fluid pathway (18) and the fluid pressure in the drug reservoir is substantially transmitted into at least a part of the fluid pathway.

Preferentially the pumping device is a pressure device (4) adapted to apply a pressure to the drug reservoir (for example on the (flexible or movable) membrane (7) of the drug reservoir (5)) in order to move the medical fluid (also called solution or drug) outside from the internal compartment of the reservoir (for example via the outlet). The pressure device may comprise a spring, a propellant chamber containing for example a liquefied gas, an expandable battery, a gas generating cell, a nitinol wire, a belleville washer, a belleville spring, Expancel beads, shape memory alloy, shape memory polymer, plunger,...

In one embodiment, the pumping device is configured to infuse the entire content of the reservoir as a single bolus.

The outlet device (10) may comprise a needle, a cannula, a tube, a connector or a transcutaneous device,...

Preferentially, the indicator device (2) comprises a body in which is arranged a cavity (13) (also called cylinder). The cavity (13) may comprise a first end (14) and a second end (15). At least one plunger (11, 12) is arranged into the cavity and is configured to move relative to the cavity. At least one plunger is configured to move depending on the pressure of the solution. In one embodiment, the indicator device indicates the infusion status thanks to a single plunger (for example via a back-and-forth motion). In another embodiment, the indicator device indicates the infusion status by using two distinct plungers (a first plunger and a second plunger).

Preferentially, the fluid pathway comprises a T-shaped channel adapted to receive the solution at a pressure substantially equal to the fluid pressure in the drug reservoir. The T-shaped channel may comprise a first fluid port in fluid communication with the outlet of the reservoir, a second fluid port in pressure communication and/or in fluid communication with the indicator device, and a third fluid port in fluid communication with an outlet device (and/or in fluid communication or in pressure communication with the valve device). The T-shaped channel is configured to provide a fluid communication from at least one of the first fluid port, the second fluid port and the third fluid port to one of the other said fluid ports.

The valve device may be configured to occlude at least one of the first fluid port, the second fluid port and the third fluid port.

The first fluid port is configured to allow the solution to flow from the internal compartment of the reservoir (from the outlet of the reservoir) to the T-shaped channel, the second fluid port is configured to be operatively coupled with the indicator device and the third fluid port is configured to allow the solution to flow from the T-shaped channel to the outlet device.

The T-shaped channel may be in fluid communication with an inlet port of the delivery device. The T-shaped channel may further comprise an additional port in fluid communication with the inlet port of the delivery device. Said inlet port of the delivery device may be configured to be in fluid communication of the internal compartment of the drug reservoir.

According to the figures 14 and 22, a part of the plunger may extend through a part of the T-shaped channel when the plunger is in a determined position (for example first position and/or third position).

The drug reservoir may comprise an inlet adapted to fill the drug reservoir with a solution to be delivered (for example if the drug reservoir is empty). The inlet may be in fluid communication with the inlet port of the delivery device. The inlet may be distinct from the outlet of the reservoir. In another embodiment, the inlet may also be the outlet of the reservoir. In this case, the outlet of the reservoir is in fluid communication with the inlet port of the delivery device.

The delivery device may comprise a housing in which the drug reservoir (5), the pumping device (22), the valve device (3), the fluid pathway (18) and/or the indicator device (2) may be arranged.

According to the figure 22a, the plunger device (11, 12) is in an initial positon which may indicate that the delivery device is ready to infuse or ready to use. This status may be the initial status of the delivery device.

If the plunger device comprises a valve device (3), when the plunger is in the initial position, the valve device may occlude the fluid pathway between the reservoir and the outlet device. This valve device may prevent the solution from reaching the outlet device before the activation of the delivery device, for example when the user fills the drug reservoir. To fill the reservoir, the delivery device may have an inlet device in fluid connection with the internal compartment of the drug reservoir. The valve device may be a rod inserted into a part of the fluid pathway and mechanically coupled to the plunger (for example the first plunger) or a part of the plunger as described below.

Focus now to the figure 22b, after an activation of the delivery device, the drug reservoir (5) is pressurized by the pressure device, the solution flows through the fluid pathway and moves the plunger device (11, 12) relative to the cavity (for example linearly (22), according to an axe defined by two ends of the cavity). The volume defined between the first plunger and the first end of the cavity increase and may receive a volume fraction of the solution. The plunger device moves toward a determined position and the indicator device indicates that the infusion is in progress. This position may be maintained until the drug reservoir is empty.

If the plunger device is in fluid communication with the solution then the cavity comprises a fluid port, and a volume fraction of the solution fills a part of the cavity (between the end comprising the fluid port and the plunger device).

If the indication device (2) comprises only one plunger then this plunger is moved. If the indicator device comprises two distinct plungers then both plungers are moved, for example, the fluid pressure moves the first plunger and the first plunger may move the second plunger (for example pushed by the first plunger or due to the compressed gas store between the first and the second plunger). During this phase, the volume between the second plunger and the second end of the cavity decreases. If the second end does not comprise any vent then the gas in this volume is compressed.

If the plunger device comprises a valve device (3), when the pressure device is activated the fluid pressure in the drug reservoir increases and reaches a determined threshold needed to open the valve device.

Focus now on the figure 22c, when the drug reservoir is emptied, the fluid pressure of the solution decreases until reaching the atmospheric pressure (or interstitial pressure if the outlet device comprises a transcutaneous device). The indicator device may be configured to allow at least one of the first plunger and the second plunger to move toward the first end in order to indicate that the infusion is over. The volume between the first plunger and the first end decreases. If this volume has received a volume fraction of the solution, the plunger motion expels the solution through the pathway to the outlet device.

The cavity may exhibit a cylindrical or quadrilateral shape.

The figure 1 shows an optional vent (40) which may be required in some embodiments.

### PRESSURIZATION MEANS AND LIQUID CONTAINER

In one embodiment, the pressure device may comprise a propellant chamber containing for example a liquefied gas having a vapor pressure at 20°C comprised in the range [+0.1 bar; +10 bar]. The pressure device may include a valve that is opened during the device activation, allowing the pressurization of the medical fluid reservoir for example by transmitting the pressure on a flexible membrane of the medical fluid reservoir.

In another embodiment, the pressurization means is a preloaded spring which acts on the drug reservoir and which is freed during the activation.

In another embodiment the reservoir is a prefilled syringe, the pressurization means being in direct communication with the plunger surface such as to transmit the pressure to the liquid for the infusion. The plunger is not pressed by the user but by a device such as an expandable battery, gas generation cell, nitinol wire, belleville washer, belleville spring, Expancel beads, shape memory alloy, shape memory polymer...

### BODY OF THE INDICATOR DEVICE

The indicator device may comprise a body having a cavity in fluid or pressure communication with a fluid pathway via a fluid port or via an element configured to transmit the pressure to the plunger arranged into the cavity.

At least a part of the indicator device may be made of a transparent plastic, e.g. polypropylene (PP), polycarbonate (PC), polyethylene (PE), polyolefin, cyclo-olefin copolymer (COC) and cyclo-olefin polymer (COP). The internal surface of the cylinder may be coated, e.g. by PEG or silicone or parylene. Lubricant (e.g. silicone oil) may also be used to improve the plunger motion. The plunger(s) is (are) made of rubber or silicone or any other hard elastomeric material. Nevertheless, the friction of the plungers against the cavity wall may prevent undesirable movement of the plunger during handling of reservoir filling.

The first variable volume may be configured to receive a volume fraction of the solution intended to be infused to the patient, thus the wall material of this volume may comprise compatible material with the solution.

### PLUNGER(S)

The plunger may comprise lips configured to slide against the cavity wall and to provide a tight interface. For example, the plunger may exhibit a structuration or sealing lips to improve the tightness and / or to make the friction dependent on the direction of move. In another embodiment one plunger can only move in one direction (as shown by the figure 4). The orientation of the lips may be comprised between +90° and -90°.

A first embodiment of the indicator device comprises a single plunger. The single plunger (also called first plunger) may be configured to perform at least one motion through the cavity and more preferentially two motions through the cavity for example a forward and backward motion. The first plunger may be configured to move depending on the pressure of the solution intended to be delivered (depending on the fluid pressure in the drug reservoir).

The single plunger may be arranged into the cavity and may define a first variable volume between the first end of the cavity and the single plunger and a second variable volume between the second end of the cavity and the single plunger.

Initially, the plunger may be at a first initial position. When the solution pressure increases (caused by the activation of the delivery), the solution pressure induces a motion of the first plunger according to a first way, until it reaches a second position. Preferentially, in this phase, the first variable volume increases and the second variable volume decreases. The second position may be maintained as long as the solution pressure is within a determined range or greater than a determined value (which may be required for the flow of the solution to the patient). When the solution pressure decreases, a biasing means (compressed gas, spring...) may move the first plunger according to a second direction (for example opposite to the first way, for example toward the first position) until a third position or the first position is reached. Preferentially, in this phase, the first variable volume decreases and the second variable volume increases.

A second embodiment of the indicator device comprises two plungers (or more, for example three). A first plunger may be configured to perform at least one motion through the cavity and more preferentially two motions through the cavity for example a forward and backward motion. The first plunger may be allowed to move depending on the pressure of the solution intended to be delivered.

The first plunger and the second plunger (and more) may be arranged into the cavity and may define a first variable volume between the first end of the cavity and the first plunger, a second variable volume between the first plunger and the second plunger, and a third variable volume between the second plunger and the second end of the cavity.

Initially, the first plunger may be in a first initial position. When the solution pressure increases (caused by the activation of the delivery), the solution pressure induces a motion of the first plunger according to a first way, to a second position. Preferentially, in this phase, the first variable volume increases, the second variable volume may stay constant or may decrease and the third variable volume may stay constant or may decrease. The second position may be maintained as long as the solution pressure is within a determined range or greater than a determined value (which may be required for the flow of the solution to the patient). When the solution pressure decreases, a biasing means (compressed gas, spring...) may move the first plunger according to a second direction (for example opposite to the first way, for example toward the first position) to a third position or the first position. Preferentially, in this phase, the first variable volume decreases, the second variable volume increases and the third variable volume stays constant. A second plunger may be configured to perform at least one motion through the cavity or two motions through the cavity for example a forward and backward motion. One motion of the second plunger may be caused by the pressure of the solution intended to be delivered and/or by the first plunger motion. For example, the solution pressure may cause a motion of the first plunger which pushes the second plunger along the same way. Initially, the second plunger may be at a first initial position. When the solution pressure increases (caused by the activation of the delivery), the solution pressure and/or the first plunger induces a motion of the second plunger according to a first direction, to a second position. The second position may be maintained as long as the solution pressure is within a determined range or greater than a determined value (which may be required for the flow of the solution to the patient). When the solution pressure decreases, a biasing means (compressed gas, spring...) may move the second plunger according to a second direction (for example opposite to the first direction, for example toward the first position) to a third position or the first position. In another embodiment, the second plunger stays in its second position even if the solution pressure decreases at the end of the delivery.

At least one plunger comprises a surface on which the fluid pressure is applied. A surface of the first plunger may be configured to be in contact with the solution to be delivered. In this case, the solution applies a force (due to the fluid pressure in the drug reservoir) on this surface causing the plunger's motion.

The second plunger (12) may comprise a a fluid pathway (such as a through hole or a porous element) which extends from one end of the second plunger to an opposite end of the second plunger for example according to an axis parallel to the axis of the elongated cavity (i.e. the axis of revolution for a cylindrical elongated cavity).

The plunger may be configured to slide against the elongated wall of the cavity. Furthermore, a sealing material (for example the plunger may comprise a sealing material) may be arranged between the plunger and the elongated wall of the cavity in order to provide a sealing interface between a volume defined downstream the plunger and another volume defined upstream the plunger.

### POSITION OF THE PLUNGER

The first plunger is configured to have at least two distinct positions: a first initial position and a second position. Preferentially, the first plunger is configured to move from the first initial position to the second position and then from the second position to the first initial position or to a third position.

The first plunger may be configured to reach the second position when the solution is flowing to the patient. The first plunger may be configured to stay at the second position over a substantial part of the duration of the solution delivery.

If the indicator device comprises two distinct plungers, then the second plunger is configured to have two distinct positions: a fourth initial position and a fifth position. The second plunger may be configured to move from the fourth initial position to the fifth position and then stay at the fifth position. The second plunger may be configured to reach the fifth position when the solution is flowing to the patient. The second plunger may be configured to stay at the fifth position after the end of the solution delivery.

### STOP MEMBER

Some figures show a stop member (23) (also called a mechanical stop). The stop member (23) is configured to stop a plunger in a determined position. This stop member may be the first end of the cavity, the second end of the cavity, may be arranged into the cavity, for example against the internal wall of the cavity or may be a magnet element (or ferromagnetic element) which cooperates with the plunger (comprising a magnet element or a ferromagnetic element) in a magnetic manner. A stop member may be dedicated to one or several plungers.

As disclosed in the figures 5, 6, 12, and 13, the stop member (23) may be configured to stop at least one plunger and to allow the motion of at least one plunger according to a single direction. Thus, the motion of at least one plunger may be restricted to only one direction by a stop member.

Some figures (see e.g. the figure 7) do not show a stop member because it may be hidden by a part of the cavity body.

The stop member may be configured to prevent a displacement of a plunger as long as a pressure threshold is not reached, as described thereafter for example in the item "INDICATOR WITH THRESHOLD EFFECT".

### ZEROING MAGNET

Initialization / zeroing of the plunger positions may be performed using a magnet placed into the cylinder. -this magnet can be used as mechanical stop. The main drawback is the loss of MRI compatibility, except if the magnet is mechanically locked in production after plunger positioning.

### CAVITY OF THE INDICATOR DEVICE

As described above, the indicator device comprises a cavity having a first end and a second end. The second end of the cavity may be connected to a communication port in fluid communication with the exterior environment of the device. This communication port may be closed (dead-end obtained using a plug or thermowelding, UV welding, over welding, US welding, overmolding ...). An initial air volume is therefore entrapped inside the cavity, for example at the atmospheric pressure, e.g. 1013 mbar absolute (1 atmosphere).

As described above, the cavity may include a first mechanical stop (23) (also called stop member) that is used to define the plunger's position during infusion (see Figure 6). After activation of the device the plunger shall reach the first mechanical stop which located in the dead-end side of the cavity, considering the worst specified pressure conditions. For instance, if the device is powered by a liquefied gas (e.g. butane), the worst conditions are met at the lowest specified temperature, which leads to the smallest value of the propellant vapour pressure and therefore the smallest value of the injection pressure *Pᵢₙⱼ*. After activation, the gas initially present in the cylinder is compressed according to the Mariotte's law. The pressure *P_{comp}* inside the volume of the cavity comprised between the first mechanical stop and the cylinder dead-end (during infusion) is function of the ratio of volume change and the temperature. It is assumed that the temperature and the pressure during the body and plungers assembly are well controlled (e.g. 1 atmosphere at 22°C). It is also assumed that the environmental conditions during the device unpacking do not modify the initial plunger positions. For a temperature operating range [+5°C; +40°C], the variability of *P_{comp}* is limited to ±6% if the first mechanical stop is reached by the plungers during infusion. This feature is important to secure the movement of the solid plunger back against a second mechanical stop at the end of the infusion, when *P_{comp}* becomes the driving pressure. This second mechanical stop formed by a cylinder conical narrowing (or an inner ring) is shown in Figures 5 and 6.

### INDICATOR WITH THRESHOLD EFFECT

The indicator may be configured so that the plunger may require a minimum pressure to initiate its displacement. Thus, for example during the filling of the drug reservoir, even if a pressure is generated (for example due to the filling), this pressure is not enough to initiate the plunger motion. It could be interesting to no change the plunger position during this phase. The initial displacement may be secured by the large pressure generated during the device activation, the pressure forces onto the plunger being much larger by design to friction forces.

The plunger may be able to reach a position after activation (right part of the cavity). During the final pressure release, when the liquid in the communication port is no longer pressurized, the plunger may move toward its initial position without reaching it due to this threshold effect.

This threshold effect may be obtained:
- *By* friction between the cylinder and the plunger:
   ∘ Friction controlled by the tolerances between the cylinder and the plunger diameters
   ∘ Friction controlled by structuration of the cylinder and / or the plunger
   ∘ Friction controlled by the presence of sealing lips (see e.g. Figure 4)
- Friction due to the inner shape of the cylinder or the outer shape of the plunger (see e.g. Figure 5)

The figures 5 show an embodiment of an infusion status indicator comprising only one plunger. The presence of a conical narrowing in the inner part of the cavity allows the definition of three different positions of the plunger:
- Initial position = plunger in the right part of the cavity
- Infusion position = plunger in the left part of the cavity
- End of infusion position = plunger in an intermediate predefined position (conical narrowing)

The figure 5d shows an example of windows arranged on the body of the indicator device that allow visualizing and therefore determining in an unambiguous way the injection status.

### FILLING PHASE

The indicator device may be configured to indicate to the user the end of the filling phase. This status may be reached when a sufficient amount of solution has been filled into the drug reservoir.

In the figures 23a to 23f is shown an indicator device (2) having an indication of the filling end, in this example the indicator device (2) further comprises four different status. A first plunger (11) (for example a solid first plunger) () and a second plunger (12) (for example a drilled plunger) are located inside the cavity. Preferentially, the delivery device (not shown) comprises furthermore a valve device (not shown) such as an anti-free flow valve having a predefined opening threshold (as described thereafter), said predefined opening threshold being substantially larger than the maximum reservoir pressure generated during the reservoir filling.

Three volumes are defined: a first variable volume between the first plunger (11) and the first end of the cavity (14), a second variable volume between both plungers (11, 12) wherein a biasing means (29) (such as a compressible spring) may be arranged (and acts on both plungers) and a third variable volume between (15) the second plunger (12) and the second end of the cavity). Optionally, the third variable volume may be vented via a venting device (28). The two plungers are initially placed on the right of a stop member (23).

If the delivery device (1) is initially empty, the user has to fill the drug reservoir (5). For this purpose, the drug reservoir may comprise an inlet port (38) in fluid communication with the internal compartment of the drug reservoir (as disclosed by the figure 1). The user may use a syringe to fill the drug reservoir (5) with the solution.

Position 1 (figure 23a): before filling, the biasing element (29) maintains the two plungers against the stop member (23) and the first end of the cavity (14). If the indicator device comprises a windows arrangement of the figure 23e, only the second plunger (12) is visible through the window 1. If the indicator device comprises the window arrangement of the figure 23f, only the first plunger (11) is visible through the window 1. In another embodiment, none of the first or second plunger may be visible through any window before filling of the reservoir.

When the user fills the drug reservoir, the solution initially stored into the syringe is moved inside the drug reservoir. The membrane (7) moves depending on the injected volume into the drug reservoir.

Position 2 (figure 23b): after filling; the first plunger (11) becomes visible through the window 2. The reservoir is designed such as to generate a slight pressure P_{fill} after filling. This pressure will move the solid plunger leftwards and compresses the spring. The stiffness of the spring is adjusted such as to allow the first plunger to reach the protrusion of the second plunger.

In order to generate a sufficient pressure into the drug reservoir during the filling (or at the end of the filling), the injected volume of solution may be greater than the volume of the internal compartment of the drug reservoir. In this case, the capacity of the internal compartment of the drug reservoir may be configured in such a manner that the filling end is characterized by a peak of pressure in the drug reservoir which allows the first plunger to reach a determined position.

Optionally, the pressure may further be generated due to the elasticity of the membrane. In this case, the elasticity of the membrane may be configured in such a manner that the filling end is characterized by a peak of pressure in the drug reservoir which allows the first plunger to reach a pre-determined position.

Position 3 (figure 23c): during infusion (after activation of the delivery), both plungers move leftwards due to the large injection pressure Pinj that is able to overcome the stop member's obstruction. If the delivery device comprises a valve device, the valve device opens the fluid pathway allowing the solution to flow to the outlet device.

If the indicator device comprises the window arrangement of the figure 23e, only the second plunger (12) is visible through the window 3. If the indicator device comprises a windows arrangement of the figure 23f, only the first plunger (11) is visible through the window 3.

Optionally, the second plunger (protrusion of the second plunger) may be configured to maintain the first plunger into a determined position during this phase and/or to prevent an over-compression of the spring. The second plunger may comprise a through hole providing a pressure equilibration or a fluid communication between the second variable volume and the third variable volume.

Position 4 (figure 23d): after infusion; the first plunger (11) is now visible through the window 4. The pressure in the cavity containing the drug drops to zero at the end of the infusion. Due to the action of the spring, the first plunger moves rightwards until reaching the stop member (23).

In this example, the figures 23e and 23f show two distinct window arrangements. The window arrangement of the figure 23e provides a set of windows allowing the first plunger and the second plunger to indicate a status of the delivery device. The window arrangement of the figure 23f provides a set of window allowing only one plunger (for example the first plunger) to indicate a status of the delivery device.

### VISUAL ELEMENT AND WINDOW INDICATOR

The indicator device may comprise a window configured to allow at least one of the first plunger and the second plunger to be visible from the outside of the body. The window is arranged in such a manner that at least one of the first plunger and the second plunger is visible in a predetermined position.

A dual visual element (for example at least two distinct plungers or with a compressible visual element) with window(s) is a robust solution since the visualization of both plungers at the end of the infusion is an assurance of the total injection of the drug.

The window(s), the visual indicator and / or the cavity (internal wall of the cavity) may be coloured to improve the injection status monitoring. The figures 7 shows an example of indicator comprising a second plunger (12) associated with a first plunger (11). Only two windows (24) are used here. Before injection no plunger (11, 12) is visible. After activation the second plunger (12) is visible. The first plunger (11) becomes finally visible after the infusion completion (the mechanical stoppers are not shown in the figures 7).

The indicator device may comprise markers to identify the infusion status. These markers may be a text, an icon, a colour, other visual means... These markers may be made by pad printing, ink jetting, or laser marking, sticker...

The indicator device may comprise a longitudinal window (24) along the cylinder to verify the plunger's positions at all time and therefore check eventual failure. This feature is notably useful in production to verify the correct initial positioning of the plungers (11, 12). This feature may be used to distinguish the initial status (no plunger visible) and the intermediate or faulty status that corresponds to the two plungers (11, 12) located in between the two vertical observation windows. The movement of the plunger may be slow (notably in case of viscous drug infusion) and therefore the time necessary to reach the first mechanical stop (also called stop member) (23) may be substantially long.

Alternative solutions may include a single window (24) with marker to identify the infusion status.

As disclosed by the figures 9, the system may include two solid plungers (11, 12) and a cylinder with both ends open. The two plungers are not initially in contact as illustrated in the figure 9a. The plunger (12) that is not in contact with the liquid to be infused moves against a mechanical stop (which may be the second end of the cavity) while the plunger in contact with the liquid moves up to an intermediate position. The compression of the air in between the plungers allows the backward motion of the plunger (11) in contact with the liquid, limiting the dead volume of the device as shown in the figures 9. This later configuration is notably useful for the placement of the plungers in production.

Three solid plungers having different colours may be used, each plunger being only visible during one predefined phase of the injection. A typical example of realisation is shown in the figures 11. In another embodiment, two plungers are used but at least one of them comprises two distinct colours.

In all embodiments the window(s) may be tinted to improve the distinction between the different infusion states.

The second plunger (12) may be substituted by a compressible visual element (31) as shown by the figures 10. In this embodiment, the indicator device may comprise a single window.

The different proposed embodiments may be combined to form alternative solution for the injection status indicator.

The window may comprise a color filter in order to change the perception of plunger color. For example, the plunger may be yellow, a first window may comprise a blue filter and a second window may comprise a magenta (or red) filter. If the plunger is located below the first window, the user sees a green color and if the plunger is located below the second window, the user sees a red (or orange)color.

### BIASING MEANS

The biasing means may be a spring, a compressible gas or an elastic element. The biasing means is used to move back the first plunger to it final position, for example from the second position to the third position or to the first position.

The previous embodiments use air as a biasing means to move back the plunger in contact with the liquid at the end of the infusion. An alternative embodiment uses a compression spring (25) located inside the cylinder to that end. A typical example is provided in the figures 12. The infusion status indicator comprises here a second plunger (12), a green plunger (11) and a compression spring (25) in between. The second plunger (12) may be drilled for initial placement purpose, so as to prevent the generation of a pressure between the plungers during the assembly. Before infusion only the second plunger (12) may be visible in the first window (24) 1. After activation the pressure of the liquid pushes the first plunger (11) and also the second plunger (12) via the compression of the spring. The infusion is on-going when the second plunger (12) is visible in the second window (24). Finally the drug pressure release at the end of the infusion induces the backward movement of the first plunger (11) which becomes visible in the third window (24). A mechanical stop (23) (conical narrowing of the cylinder) may be used to secure the final position of the first plunger after infusion.

An alternative embodiment may comprise a single spring (25) and plunger (11) inside a cylinder with one stop limiter (23) as illustrated in the figures 13. The mechanism is similar to the one described in the figures 5, through the restoring force that is used for the backward movement of the plunger after infusion is no longer compressed air but a compression spring. The plunger can be seen iteratively in each of the 3 windows shown in the figure 13d, depending on the infusion status (ready to inject, injection on going and end of infusion).

### ANTI-FREE FLOW VALVE

Reservoir filling, whatever the nature of the reservoir itself, may pressure the drug and generate a free-flow. To prevent this undesirable flow (free flow) of drug (which may be problematic if the filling is made before the placement of the device onto the patient and the introduction of a cannula or a needle into the patient's skin), it is first proposed to use the plunger (11) in contact with the liquid as a valve device. The figure 14 shows an example of plunger having a protrusion or a shape (a conical shape) which allows blocking the flow as long as the pressure of the drug does not exceed a predefined value (which may depend on plunger friction against the inner wall of the cylinder and on the surface of the plunger that is submitted to the drug reservoir pressure after activation). At the end of the infusion the valve may be again in closed position if no mechanical stop is used (see e.g. figures 5 for a typical example of mechanical stop for the backward movement of the plunger).

Focus on the figure 14, the T-shaped channel as described above comprises a third fluid port in fluid communication with the outlet device (for example to the needle). This third fluid port may exhibit a conical shape to improve the tightness of this valve at low pressure.

Such a valve is an alternative to classical solution to block the flow at low pressure, including the use of a hydrophobic porous media, a mitral valve...between the reservoir and the needle.

An alternative embodiment exhibits a needle that is off-axis of the cylinder as shown in the figure 15. The schematic cross-section of the device is shown except for the off-axis needle that is entirely represented for sake of clarity. The solid plunger (11) that is intended to be in contact with the liquid blocks the flow from the reservoir to the needle until the drug pressure exceeds a predefined threshold value.

The figure 15 shows a fluid pathway without a T-Shaped channel. The first end of the cavity comprises:
- a first fluid port providing a fluid communication between the outlet of the reservoir and the cavity and acts as an inlet, and
- a second fluid port providing a fluid communication between the cavity and the outlet device and acts as an outlet,
The fluid pathway is divided in two parts: a first part upstream of the cavity and a second part downstream of the cavity.

The indicator device is further used as a valve device which closes the fluid pathway when the fluid pressure is lower than a determined threshold. The valve may be closed before the activation of the delivery and after the end of the infusion, thanks to the forward and backward motion of the first plunger.

### ANTI-FREE-FLOW VALVE WITH CONTROLLED OPENING THRESHOLD

The figure 16 shows another embodiment of the present invention. This device is similar to the one disclosed in the figure 1 except for the presence of a valve device (3) (also called valve) in front of the needle inlet. This valve may comprise an additional (movable)plunger (32), a valve cavity (37) initially filled with compressible gas (for example air), and/or a plug (27). At rest (before activation of the device), the top surface of the plunger (32) is partly submitted to the pressure inside the communication chamber since mechanical stops (26) (also called plunger stoppers) are used to that end. This plunger itself may exhibit a structuration or texturizing that also leads to the same effect. During assembly, the introduction of the plug leads to the compression of the air comprised inside the cavity. It becomes possible to adjust the pretension (or opening threshold) of the valve by controlling by design the pressure inside the cavity in production, e.g. by introducing a plug having a well-controlled volume such as to generate always the same initial pressure inside the cavity. The activation of the device leads to the generation of a large pressure in the reservoir. This pressure will push against the top surface of the second solid plunger and will move it perpendicularly to the direction of the needle, freeing the fluid pathway through the needle. At the end of the infusion the second plunger will close again the inlet of the needle.

The opening threshold is typically larger than the maximum pressure that can be generated during filling in normal situation. The opening threshold may be adjustable using a threaded plug that is more or less screwed according to the target value of the valve pretension.

An alternative embodiment comprises a compression spring inside the cavity, said cavity being vented. The pretension may again be adjusted by controlling the initial spring compression, e.g. using a threaded plug that will used to control the initial length of the compression spring, as shown in the figure 17 wherein the plug is also vented.

### CRACK VALVE

In another embodiment, the communication chamber comprises a flexible membrane in front of the inlet of a needle having both ends bevelled as shown in the figure 18. The flexible membrane will be pierced by the needle if the reservoir pressure exceeds a predefined value, freeing the fluidic pathway for the drug. The membrane is made of an elastomeric material to allow a large deflection and prevent a crack before the piercing by the needle.

In another embodiment the membrane cracks by the sole application of the pressure (mechanical failure due to the excess of strain induced by the large applied pressure after activation), the needle having a single bevel at the outlet (see the figure 19).

In another embodiment the valve is a plunger (32). As shown in the figure 20, a solid plunger (32) is placed in a second cylinder in direct communication with the needle inlet and the communication port. The activation of the device leads to the piercing of the plunger by the needle as illustrated in the figure 21. The plunger may be partially introduced into the needle in production to limit the dead volume of the device by reducing the size of the volume between the plunger (of the valve) and the needle). Moreover, it also prevents the injection of a large air volume in the patient at the beginning of the injection. As a compressible gas volume may be trapped in the valve cavity (37) at the end of the infusion, the plunger may move toward it initial position and block the flow. The compressible gas may be substituted by a biasing element such as a spring...

## Claims

1. An indicator device for indicating an infusion status of a medical device adapted to deliver a solution to a patient, the indicator device including:
A body,
An elongated cavity arranged into the body and having a first end and a second end;
A first plunger configured to move relative to the elongated cavity; and
A second plunger configured to move relative to the elongated cavity Wherein the first plunger and the second plunger are arranged into the elongated cavity in such a manner to define:
- a first variable volume formed between the first end of the elongated cavity and the first plunger,
- a second variable volume formed between the first plunger and the second plunger, and
- a third variable volume formed between the second plunger and the second end of the elongated cavity.
Wherein the first plunger moves depending on the pressure of the solution intended to be delivered.

2. Indicator device according to the claim 1, wherein the first plunger is in pressure communication with the solution intended to be delivered.

3. Indicator device according to one of the previous claims further comprising a fluid port in fluid communication with the solution intended to be delivered.

4. Indicator device according to one of the previous claims, wherein the second plunger comprises a pathway configured to provide a fluid communication between the second variable volume and the third variable volume.

5. Indicator device according to one of the previous claims, wherein the elongated cavity further comprises a stop member arranged between the first end and the second end in order to maintain at least one of the first plunger and the second plunger in a determined position.

6. Indicator device according to one of the previous claims, wherein the second end comprises a vent in order to provide a pressure equilibration of the third variable volume with the outside environment.

7. Indicator device according to one of the previous claims further comprising a biasing means arranged between the first plunger and the second end or between the first plunger and the second plunger.

8. Indicator device according to one of the previous claims, wherein the first variable volume is in pressure communication with a pressurized reservoir storing the solution intended to be delivered.

9. An indicator device for indicating an infusion status of a medical device adapted to deliver a solution to a patient, the indicator device including:
A body,
An elongated cavity arranged into the body and having a first end and a second end; and
A first plunger configured to move relative to the elongated cavity;
Wherein the first plunger is arranged into the elongated cavity dividing the cavity in two variable volumes: a first variable volume and a second variable volume
Wherein the first plunger is configured to have three distinct positions:
- a first initial position,
- a second position, and
- a third position
Wherein the first plunger moves depending on the pressure of the solution intended to be delivered.

10. Indicator device according to the claim 9 further comprising a second plunger configured to move relative to the elongated cavity, wherein the second plunger moves through the second variable volume.

11. Indicator device according to the claim 10, wherein the second plunger comprises a through hole.

12. Indicator device according to one of the previous claims 9 to 11, wherein the first volume is in pressure communication with a pressurized reservoir storing the solution intended to be delivered.

13. A medical device adapted to deliver a solution to a patient, the medical device including:
An indicator device according to the claim 1 or 9,
A pressurized reservoir for storing the solution,
An outlet port in fluid communication with the patient
A fluid pathway from the interior of the pressurized reservoir to the outlet port
Wherein the indicator device is in pressure communication with the fluid pathway.

14. Medical device according to the claim 13, wherein the indicator device further comprises a valve device configured to prevent the solution from reaching the outlet port.

15. Medical device according to the claim 14, wherein the valve device is a part of the first plunger which occludes the fluid pathway when the first plunger is in a predetermined position.
